# EUROPEAN PATENT APPLICATION

(11) **EP 2 025 341 A1**
(43) Date of publication of application: **18.02.2009**
(21) Application number: 08380026.8
(22) Date of filing: 04.02.2008
(51) Int. Cl.: A61K 36/53, A61K 36/63, A61K 36/515, A61K 31/57, A61P 17/00, A61K 31/305

(54) **Ointment for combating and treating skin diseases, and process for preparing said ointment**

(30) Priority: 13.08.2007 ES 200702275
(71) Applicant: Ramos Prieto, José, 18330 Chauchina Granada (ES)
(72) Inventor: Ramos Prieto, José, 18330 Chauchina Granada (ES)
(74) Representative: Gonzalez Gonzalez, Pablo

(57) **Abstract**

The invention relates to an ointment for treating and curing skin diseases of human beings such as psoriasis, dermatitis, acne, herpes and fungi, consisting of a composition obtained based on ingredients such as white petrolatum, , clobetasol propionate, distilled water, rosemary honey, virgin olive oil, white precipitate mercury chloride, salicylic acid, and gentian violet.

It also describes a process for preparing said ointment basically consisting of preparing a completely homogenized composition from the mentioned ingredients with the use of a stirring device for a time of about 30 minutes and maintaining the temperature at around 20°C in order to obtain the cream or ointment appearance of said composition.

## Description

### Object of the Invention

The present invention relates to an ointment for combating and treating skin diseases and to a process for preparing said ointment, providing essential novel characteristics and considerable advantages with regard to the products known and used in the current state of the art for identical or equivalent applications.

The invention particularly proposes developing a product especially indicated for treating certain skin diseases in humans, some of which have been difficult to treat and cure up until now such as psoriasis, dermatitis, acne, herpes and fungi, by directly applying the product on the affected area. Similarly, a process for preparing the ointment has also been developed, which is simple to design and easy to carry out without needing to establish specific complex implementation conditions and without needing complicated and costly equipment.

The application field of the invention is generally comprised within the medical sector and more specifically in the field of producing preparations for treating human skin diseases.

### Background and Summary of the Invention

The existence of a wide variety of skin conditions the treatment of which can be more or less difficult according to the specific condition in question is generally known by all. Some of those diseases are more or less common among the human population, such as the case of acne, dermatitis, herpes or fungi which at certain moments can spread, to a lesser or greater extent, by contagion or in another manner, and others are of a more complex type, not contagious but without a specific treatment allowing the definitive cure thereof up until now, as occurs with psoriasis for example.

Although they are normally chronic conditions which are mostly usually caused by viruses, in the case of psoriasis there is no certain knowledge of its origin and it is associated with certain factors that are both hereditary and associated to the environment of the people suffering from it. In all considered cases, they are annoying diseases which negatively affect the daily life of many people suffering from them.

Of course, multiple remedies are known in the state of the art which in some cases completely solve the problem, and in other cases considerably lessen it.

Taking into consideration the characteristics of these types of conditions and the high overall number of people suffering from them, the present invention has proposed as its main objective the preparation of a product which, applied to the areas affected by any of said diseases, allows completely curing the disease, including psoriasis, after a certain period of time. This object has been fully reached by means of the curative product which will be object of the description below, the main characteristics of which are included in the characterizing part of attached claim 1.

The invention has likewise developed a process for preparing said curative product, the main characteristics of which are included in the characterizing portion of attached claim 2.

The curative product developed and tested by the present invention has essentially been prepared from ingredients which are mostly generated naturally and which, once combined with one another in the conditions which will be detailed below, allow obtaining a final product in the form of an ointment, which applied to the affected area allows curing the condition, together with eliminating the itching or flaking symptoms which are normally associated to the previously mentioned type of diseases.

The preparation process in turn starts from the formation of a mixture unified and prepared by means of an ingredient combination phase in predetermined conditions.

### Description of a Preferred Embodiment

As has been previously mentioned, a first object of the invention consists of preparing a product, preferably in the form of an ointment, which applied to human skin affected by diseases such as psoriasis, dermatitis, acne, herpes and fungi, allows eliminating the problem and curing the person affected by any of such diseases.

According to another aspect of the invention, a second objective consists of developing a process which allows obtaining the mentioned product for treating and curing the disease.

In a preferred embodiment, the curative product consists of a compound prepared from the ingredients mentioned below in the expressed amounts:

| | |
|---|---|
| White Petrolatum | 1,250.00 g |
| Clobetasol Propionate | 0.50 g |
| Distilled Water | 0.50 g |
| Rosemary Honey | 90.00 g |
| Virgin Olive Oil | 30.00 g |
| White Precipitate Mercury Chloride | 84.00 g |
| Salicylic Acid | 84.00 g |
| Gentian Violet | 5.00 g |

The compound obtained has the appearance of an ointment, although this fact must not be interpreted as limiting given that it could also be prepared in the form of a cream, if desired.

As can be seen above, the amounts are expressed by weight, for batch preparation, and correspond to those that would be necessary for preparing each unit batch.

The curative product obtained as a result of the previous composition is prepared based on a process comprising the following phases:
- Preparing a mixture by combining all the ingredients which are involved in preparing the product, mentioned above;
- Arranging the mixture thus formed inside a stirring device, of an industrial mixer type or of a type stirring the ingredients with sufficient assurances of a complete and intimate mixing action therebetween;
- Maintaining the stirring for a period of time of about 30', while the temperature is controlled at a value close to 20°C, and
- Verifying the uniformity of the composition and removing it.

The temperature of around 20°C which was mentioned as the most suitable one for preparing the curative product of the invention has proved to be the most ideal temperature due to the fact that the petrolatum ingredient melts at approximately this value and provides the final composition with the desired cream or ointment appearance so that the product can be easily applied by the user. The ointment will be applied on the affected area according to the frequency suggested by the particular conditions and circumstances of each patient.

It is not considered necessary to make the content of the present description more extensive so that a person skilled in the art can understand its scope and the advantages derived therefrom as well as carry out its practical implementation.

In spite of the foregoing, and given that the description only corresponds to a preferred embodiment, changes and modifications can be foreseen without this altering the essence of the invention, which can affect both changes in the values of each ingredient according to certain particular needs, or also the possible inclusion of other auxiliary ingredients allowing concrete applicative specificities of the product obtained.

## Claims

1. An ointment for combating and treating skin diseases, particularly for treating and curing skin diseases of human beings, such as psoriasis, dermatitis, acne, herpes and fungi, **characterized in that** it consists of a composition according to the following respective ingredients and amounts:
| | |
|---|---|
| White Petrolatum | 1,250.00 g |
| Clobetasol Propionate | 0.50 g |
| Distilled Water | 0.50 g |
| Rosemary Honey | 90.00 g |
| Virgin Olive Oil | 30.00 g |
| White Precipitate Mercury Chloride | 84.00 g |
| Salicylic Acid | 84.00 g |
| Gentian Violet | 5.00 g |
the composition being able to be directly applied on the affected area and according to predetermined applicative frequencies.

2. A process for preparing an ointment consisting of the composition of claim 1, **characterized in that** it comprises the following phases:
- Preparing a mixture by combining all the ingredients which are involved in preparing the product, mentioned above;
- Arranging the mixture thus formed inside a stirring device, of an industrial mixer type or of a type stirring the ingredients with sufficiently assurances of a complete and intimate mixing action therebetween;
- Maintaining the stirring for a period of time of about 30', while the temperature is controlled at a value close to 20°C, and
- Verifying the uniformity of the composition and removing it.
